# EUROPEAN PATENT APPLICATION

(11) **EP 3 158 978 A1**
(43) Date of publication of application: **26.04.2017**
(21) Application number: 15809933.3
(22) Date of filing: 12.05.2015
(51) Int. Cl.: A61F 2/89, A61F 2/90, A61L 27/58, A61L 27/54

(54) **STENT FOR CONFLUENT BLOOD VESSEL**

(30) Priority: 19.06.2014 KR 20140074780
(71) Applicant: M.I.Tech Co., Ltd., Pyeongtaek-si, Gyeonggi-do 451-864 (KR); Industry-Academic Cooperation Foundation, Yonsei University, Seoul 120-749 (KR)
(72) Inventor: KWON, Hyuck Moon, Seoul 06273 (KR); HAN, Jong Hyeon, Seoul 06603 (KR); PARK, Hun Kuk, Pyeongtaek-si Gyeonggi-do 450-731 (KR); JANG, Bong Seok, Osan-si Gyeonggi-do 447-719 (KR); YUN, Ho, Asan-si Chungcheongnam-do 336-873 (KR)
(74) Representative: dompatent von Kreisler Selting Werner - Partnerschaft von Patent- und Rechtsanwälten mbB
(86) International application number: PCT/KR2015/004719
(87) International publication number: WO 2015/194759

(57) **Abstract**

A stent for a confluent blood vessel of the present invention comprises: a plurality of first wires which consist of a number of rows in one direction and of which the shape is maintained inside a human body; a plurality of second wires which intersect with the first wires and are formed as a number of rows; and a connector which connects the portion where the first wires and the second wires intersect with each other, wherein a plurality of hollows are formed by the first wires and the second wires, and at least one of the plurality of second wires is decomposed after a predetermined time inside the human body. Accordingly, the following effects are anticipated: when removing the stent, removal is easy because the second wires consisting of the biodegradable polymer are gradually decomposed by bodily fluids; and a stent insertion effect can be expected to be sustained for a long time because the shape of the stent is maintained over a predetermined time. In addition, as drugs are sequentially released during the decomposing process of the second wires, there is an effect of drugs being continuously released.

## Description

### Technical Field

The present invention relates to a hybrid stent for a blood vessel.

### Background Art

Among blood vessel diseases, angiostenosis refers to a disease in which the diameter of a peripheral blood vessel is narrowed to less than 50% of that of a normal blood vessel due to a cause, such as hyperlipidemia, obesity, smoking, stress, lack of exercise, diabetes, or the like.

As a result, a blood circulation disorder may occur, and thus a phenomenon in which legs are asleep, muscle pain or the like may be caused. When an ischemic ulcer develops, a corresponding region may need to be cut away.

Accordingly, in order to treat the disease, angioplasty designed to insert a tool, such as a stent, into a narrowed blood vessel and expand the blood vessel is performed.

Generally, stents are medical tools that are inserted into narrowed regions and expand and maintain narrowed tubes to and at their original sizes. Recently, there has been a tendency for stents to be used for the treatment of cerebrovascular diseases, such as a brain aneurysm and the like.

Although such stents reduce the risk of stenosis of renewed blood vessels, they cannot completely prevent restenosis.

After stent implantation surgery, the surface of a stent is in direct contact with blood. Serious thrombosis that causes restenosis of a blood vessel may occur due to an external surface. In a considerable number of patients, restenosis of blood vessels occurs due to neointimal hyperplasia within the inner diameter of the stent.

Accordingly, research into drugs that suppress restenosis is being actively carried out. Although various types of drugs have produced desirable results, a high concentration or large amount of drug must be administered to the entire body in order to obtain the effects of the drugs, with the result that the risk of producing a side effect has been pointed out.

This problem can be overcome by coating the surface of a stent with a drug to locally transfer the drug to a lesion region and, thus, neointimal hyperplasia can be suppressed and also the problem of restenosis of a blood vessel can be overcome.

However, hybrid stents for a blood vessel, which are clinically used, are all made of metal. Since a metallic material does not allow a drug to be mixed therewith, a drug must be attached onto the surface of the material through coating or the like. This technology includes a method of coating or adhering a drug, as described in Korean Patent Application Publication No. 10-2004-7005561.

However, when a drug is attached onto the surface of a metal stent by the coating or adhesion method, a problem arises in that the drug itself is peeled away and separated from the surface of the stent. Furthermore, there are difficulties in ensuring an appropriate period of time and attaching an appropriate amount of drug in order to obtain sufficient medicinal effect.

### Disclosure

### Technical Problem

Accordingly, a hybrid stent for a blood vessel according to the present invention is intended to overcome the above-described problems, and an object of the present invention is to provide a stent that enables an appropriate amount of drug to be eluted within a region, into which the stent has been inserted, over an appropriate period of time.

A further object of the present invention is to enable part of a stent made of a biodegradable polymer to be gradually dissolved and eliminated inside a human body (biodegradable vascular scaffolding) and to include a drug inside the biodegradable polymer to locally and continuously elute the drug in a narrowed region of a blood vessel (elution of a drug), in order to minimize the development of restenosis.

### Technical Solution

In order to accomplish the above objects, the present invention provides a hybrid stent for a blood vessel, the stent including: a plurality of first wires configured to form a plurality of rows in one direction and maintain the shape thereof inside a human body; a plurality of second wires configured to intersect the first wires and form a plurality of rows; and connectors configured to connect portions where the first wires and the second wires intersect each other; wherein a plurality of hollow holes are formed by the first wires and the second wires, and one or more of the plurality of second wires are degraded inside the human body after a predetermined period of time.

The second wires may be each formed by mixing or coating a biodegradable polymer, degradable inside the human body after a predetermined period of time, with at least one type of drug.

The biodegradable polymer may include at least any one of polyvinyl alcohol, polyethylene glycol, polylactide, polyglycolide, polyethylene oxide, polydioxanone, polycaprolactone, polyphosphazene, polyanhydride, polyamino acid, cellulose acetate butylate, cellulose triacetate, polyacrylate, polyacrylamide, polyurethane, polysiloxane, and polyvinylpyrrolidone.

The drug may include at least one of an anti-cancer drug including a paclitaxel-based drug, an immunesuppressive drug including a sirolimus- or limus-based drug, and an antiplatelet drug including cilostazol.

Each of the second wires may include: a body portion made of a first biodegradable polymer; and a coating layer made of a mixture of a second biodegradable polymer having a composition identical to or different from that of the first biodegradable polymer and the drug, and coated on the outer circumferential surface of the body portion.

### Advantageous Effects

According to the present invention described above, the following effects are achieved:
First, the effect of expanding and maintaining a blood vessel by using the first wires made of a shape-maintaining metal can be expected because the second wires made of the biodegradable polymer are gradually degraded by body fluids after surgery for the implantation of the stent, and also the effect of preventing restenosis of the blood vessel can be expected because the drug included in the second wires made of the biodegradable polymer is continuously discharged.
Second, the effect of maintaining a stent insertion effect for a prolonged period of time can be expected because the shape of the stent can be maintained even after a predetermined period of time.
Third, the effect of sufficiently suppressing restenosis of a blood vessel attributable to neointimal hyperplasia can be achieved because the drug is gradually discharged during degradation of the second wires and thus the effect of continuously discharging the drug is achieved.

### Description of Drawings

FIG. 1 is a view showing a hybrid stent for a blood vessel according to the present invention;
FIG. 2 is a sectional view showing the structure of a second wire according to an embodiment of the present invention;
FIG. 3 is a sectional view showing the structure of a second wire according to another embodiment of the present invention; and
FIG. 4 is a flowchart showing a process of manufacturing a hybrid stent for a blood vessel according to the present invention.

### Mode for Invention

Preferred embodiments of the present invention will be described in greater detail with reference to the accompanying drawings. Well-known technical parts will be omitted or abridged for brevity of description.

The preferred embodiments of the present invention are described in greater detail below with reference to the accompanying drawings.

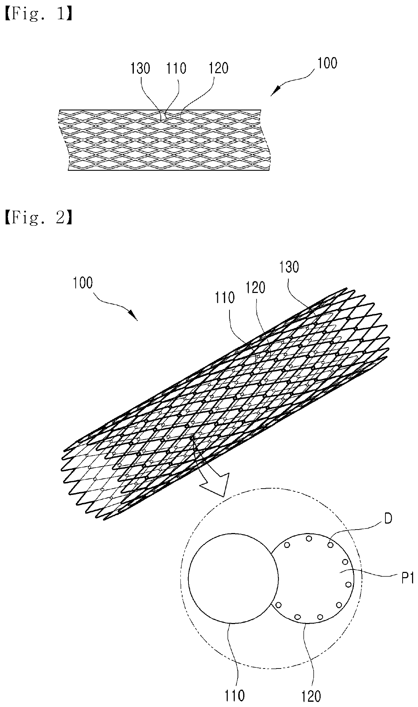

Referring to FIG. 1, a hybrid stent 100 for a blood vessel according to an embodiment of the present invention is provided in a hollow cylindrical shape. The hybrid stent 100 for a blood vessel according to the present invention is configured to include first wires 110, second wires 120, and connectors 130.

The first wires 110 are formed in a plurality of rows of the hybrid stent 100 for a blood vessel to extend in a helical direction along a circumferential direction.

In particular, the first wires 110 are made of a shape-memory alloy material, such as a nickel-titanium alloy. The first wires 110 may expand or contract in directions toward the circumferences thereof under a specific condition, such as a specific temperature or the like.

That is, the first wires 110 are inserted into and placed in a lesion region of a blood vessel, expand in directions toward the inner wall of the blood vessel, and support the inner wall of the blood vessel, thereby preventing restenosis of the blood vessel.

Accordingly, the shape of the hybrid stent 100 for a blood vessel is maintained inside a lumen or blood vessel of a human body by bending moment. The second wires 120 are formed in a plurality of rows to extend in a helical direction along a circumferential direction so as to intersect the first wires 110. The first wires 110 and the second wires 120 intersect each other, and thus a plurality of fine hollow holes are formed.

The size of the hollow holes varies depending on a region into which the hybrid stent 100 for a blood vessel is inserted.

The connectors 130 are components that connect and fasten portions where the first wires 110 and the second wires 120 intersect each other.

One or more of the plurality of second wires 120 are made of a biodegradable polymer that is degraded by body fluids after a predetermined period of time.

In this case, the biodegradable polymer includes, but is not limited to, polyvalerolactone, poly-ε-decalactone, polylactonic acid, polyglycolic acid, polylactides, polyglycolides, copolymers of polylactides and polyglycolides, poly-ε-caprolactone, polyhydroxybutanoic acid, polyhydroxybutyrates, polyhydroxyvalerates, polyhydroxybutyrate co-deoxanones, polyanhydrides such as polymaleic acid anhydride, polycyanoacrylates, polycaprolactone dimethyl acrylates, poly-b-maleic acid, polycaprolactone butyl acrylates, multiblock polymers such as polymers of oligocaprolactonedioles and oligodioxanonedioles, polyetherester multiblock polymers such as PEG and poly(butyleneterephthalates), polypivotolactones, polyglycolic acid trimethyl-carbonates, polycaprolactone-glycolides, poly(g-ethylglutamate), poly(DTH-iminocarbonate), poly(DTE-co-DT-carbonate), poly(bisphenol A-iminocarbonate), polyorthoesters, polyglycolic acid trimethylene carbonates, polytrimethylene carbonates, polyiminocarbonates, poly(N-vinyl)pyrrolidone, polyvinyl alcoholes, polyester amides, glycosylated polyesters, polyphosphoesters, polyphosphazenes, poly[(p-carboxyphenoxy)propane], polyhydroxypentanoic acid, polyanhydrides, polyethylene oxide-propylene oxide, soft polyurethanes, polyurethanes with amino acid moieties in the backbone, polyether esters such as polyethylene oxide, polyalkene oxalates, polyorthoesters, and their copolymers, lipids, carrageenans, fibrinogen, starch, collagen, protein-based polymers, polyamino acids, synthetic polyamino acids, zein, modified zein, polyhydroxyalkanoates, pectic acid, actinic acid, modified and unmodified fibrin and casein, carboxymethyl sulfate, albumin, hyaluronic acid, heparan sulfate, heparins, chondroitin sulfate, dextran, b-cyclodextrins, copolymers with PEG and polypropylene glycol, gum arabicum, guar, gelatins, collagen, collagen-N-hydroxysuccinimide, lipids, phospholipids, modifications and copolymers and/or mixtures of the aforementioned substances, which may have a degradation period varying depending on the type of composition and composition ratio.

A drug D includes at least one of an anti-cancer drug including a paclitaxel-based drug, an immunosuppressive drug including a sirolimus- or limus-based drug, and an antiplatelet drug including cilostazol.

The second wires 120 of the present invention are each formed by mixing a biodegradable polymer, degradable after a predetermined period of time, with at least one type of drug D. The structures of the second wires 120 according to the present invention are described based on respective embodiments.

### <First embodiment>

Referring to FIG. 2, it can be seen that the second wires 120 are each formed by mixing a first biodegradable polymer P1 with a drug D.

When the first biodegradable polymer P1 is degraded by body fluids, the drug D mixed with the first biodegradable polymer P1 in advance is discharged.

### <Second embodiment>

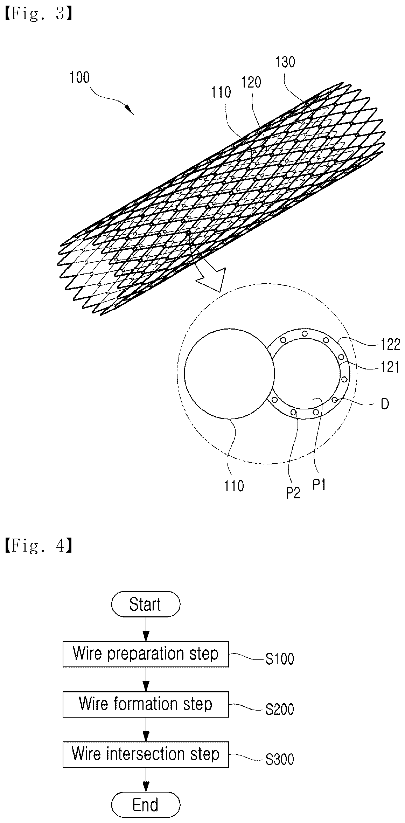

Referring to FIG. 3, the second wires 120 are each configured to include a body portion 121 and a coating layer 122 coated on the outer circumferential surface of the body portion 121.

The body portion 121 is made of the first biodegradable polymer P1.

The coating layer 122 is formed by coating a second biodegradable polymer P2 on the outer circumferential surface of the body portion 121. The second biodegradable polymer P2 includes a drug D.

In this case, although the first biodegradable polymer P1 and the second biodegradable polymer P2 may be the same biodegradable polymer, the first biodegradable polymer P1 and the second biodegradable polymer P2 may be different materials depending on the type of drug D, a drug elution period, an elution location, or the like.

### <Third embodiment>

There are cases where a long drug discharge period and a plurality of drugs are required in order to prevent restenosis.

In these cases, a drug D' different from the drug D included in the second biodegradable polymer P2 may be included in the first biodegradable polymer P1.

Accordingly, after the second biodegradable polymer P2 has been degraded, the different drug D' included in the first biodegradable polymer P1 is discharged while the first biodegradable polymer P1 is biodegraded.

Therefore, a plurality of coating layers 122 may be stacked in each of the second wires 120 depending on a different elution interval or period of the drug D, the type of drug D, or the like.

The periods over which drugs D are discharged may be adjusted by varying the compositions and compositional proportions of biodegradable polymers P1, P2, P3,..., Pn constituting the respective coating layers 122, with the result that the effect of sequentially providing the drugs D required for a lesion region can be expected.

### <Description of a method of manufacturing the hybrid stent 100 for a blood vessel>

A method of manufacturing the stent of the first embodiment, i.e., an embodiment of the hybrid stent 100 for a blood vessel, according to the present invention will be described. Redundant descriptions will be omitted because they have been already given above.

### First step: wire preparation step <S100>

In a first step, methods of including a drug in the second wires 120 according to the present invention may be basically classified into the following three types:
1) A method of directly attaching a drug into a metal stent
2) A method of loading a drug into pores of a porous metal stent
3) A method of mixing a polymer, coated on a stent, with a drug
4) A method of dissolving a drug in a biodegradable polymer and eluting the drug as the polymer is biodegraded

In the present embodiment, the second wires 120 are prepared by including drugs D in biodegradable polymers P1, P2,... by using the third or fourth method.

### Second step: wire formation step <S200>

The first wires 110 may be made of a hyperelastic shape-memory alloy material, may be manufactured within a case, tube or mold having a desired shape, and may be laser- cut to have an appropriate structure, pattern or shape, or a combination thereof, or be laser-cut into an appropriate structure, pattern or shape, or a combination thereof.

Furthermore, the first wires 110 may be polymerized within a mold having a specific structure, pattern or shape, or a combination thereof.

In practice, during or after a manufacturing process, a desired pattern may be etched in or on a tube, or the first wires 110 may be machined or laser-bent.

In an implementation, the formation of the first wires 110 using a three-dimensional (3D) printer may be also considered.

This case is advantageous in that the thickness, length, bending, shape or the like of the first wires 110 may be customized to be suitable for a bodily region of a patient, whom the stent will be inserted into, and to have a maximum effect.

In the case of the second wires 120, wires are sprayed via a wire spraying apparatus by using the wires prepared in the first step S100.

The biodegradable polymer P1 including the drug D is sprayed onto a mandrel in accordance with a predetermined movement pattern of the mandrel including the rectilinear movement of the mandrel along a lengthwise direction axis and additionally including the rotating movement of the mandrel around the lengthwise direction axis.

After the first wires 110 and the second wires 120 have been formed, the step of coating the wires 110 and 120 may be further included.

### Third step: wire intersection step <S300>

In a third step S300, the step of intersecting the first wires 110 and the second wires 120 with each other at different locations in a circumferential direction is performed.

Connectors 130 that are contractible in a lengthwise direction are formed in such a manner that the first wires 110 and the second wires 120 intersect each other in downward and upward directions and are hooked to each other at different locations. Diamond-shaped hollow holes are formed among the connectors 130 in the same circumferential and lengthwise directions, finally forming a cylindrical body.

The first wires 110 and the second wires 120 are combined together in a form in which they can freely perform the operations of contracting and expanding but cannot be separated from each other, thereby forming the hybrid stent 100 for a blood vessel.

The embodiments disclosed herein are not intended to limit the technical spirit of the present invention, but are intended to illustrate the technical spirit of the present invention. The scope of the technical spirit of the present invention is not limited by these embodiments. The range of protection of the present invention should be defined based on the following claims. All technical spirits that fall within an equivalent range should be interpreted as being included in the range of rights of the present invention.

### (Description of reference symbols)

| | | | |
|---|---|---|---|
| 100: | hybrid stent for a blood vessel | | |
| 110: | first wire | | |
| 120: | second wire | | |
| 121: | body portion | 122: | coating layer |
| P1: | first biodegradable polymer | | |
| P2: | second biodegradable polymer | | |
| D: | drug | D' : | different drug |
| 130: | connector | | |

## Claims

1. A hybrid stent for a blood vessel, the stent comprising:
a plurality of first wires configured to form a plurality of rows in one direction and maintain a shape thereof inside a human body;
a plurality of second wires configured to intersect the first wires and form a plurality of rows; and
connectors configured to connect portions where the first wires and the second wires intersect each other;
wherein a plurality of hollow holes are formed by the first wires and the second wires, and one or more of the plurality of second wires are degraded inside the human body after a predetermined period of time.

2. The stent of claim 1, wherein the second wires are each formed by mixing a biodegradable polymer, degradable inside the human body after a predetermined period of time, with at least one type of drug.

3. The stent of claim 2, wherein the biodegradable polymer comprises at least any one of polyvinyl alcohol, polyethylene glycol, polylactide, polyglycolide, polyethylene oxide, polydioxanone, polycaprolactone, polyphosphazene, polyanhydride, polyamino acid, cellulose acetate butylate, cellulose triacetate, polyacrylate, polyacrylamide, polyurethane, polysiloxane, and polyvinylpyrrolidone.

4. The stent of claim 2, wherein the drug comprises at least one of an anti-cancer drug including a paclitaxel-based drug, an immunesuppressive drug including a sirolimus- or limus-based drug, and an antiplatelet drug including cilostazol.

5. The stent of claim 2, wherein each of the second wires comprises:
a body portion made of a first biodegradable polymer; and
a coating layer made of a mixture of a second biodegradable polymer having a composition identical to or different from that of the first biodegradable polymer and the drug, and coated on an outer circumferential surface of the body portion.
